## Europäisches Patentamt

## European Patent Office

## Office européen des brevets

(11) Veröffentlichungsnummer: **0 100 070**
**B1**

(12) **EUROPÄISCHE PATENTSCHRIFT**

(45) Veröffentlichungstag der Patentschrift:
**12.04.89**

(51) Int. Cl.⁴: **A 61 K 33/08**

(21) Anmeldenummer: **83107188.1**

(22) Anmeldetag: **22.07.83**

(54) **Antacida sowie Verwendung von zeolithischen Molekularsieben zur Herstellung von Antacida und bei der Bekämpfung von Hyperacidität.**

(30) Priorität: **30.07.82 DE 3228485**

(43) Veröffentlichungstag der Anmeldung:
**08.02.84 Patentblatt 84/6**

(45) Bekanntmachung des Hinweises auf die Patenterteilung:
**12.04.89 Patentblatt 89/15**

(84) Benannte Vertragsstaaten:
**AT BE CH DE FR GB IT LI LU NL SE**

(56) Entgegenhaltungen:
**DE-A- 2 029 265**
**JP-A-53 020 437**
**US-A- 4 379 143**

**J. Schnekenburger, In-vitro-Untersuchungen an Antacida I-III**
**CHEMICAL ABSTRACTS, Band 99, Nr. 6, 8. August 1983, p. 323, Zusammenfassung 43570x, Columbus, Ohio, US**

**Die Akte enthält technische Angaben, die nach dem Eingang der Anmeldung eingereicht wurden und die nicht in dieser Patentschrift enthalten sind.**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Greb, Wolfgang, Dr., Dr., Alte Landstrasse 48, D-4000 Düsseldorf (DE)**
Erfinder: **Christophliemk, Peter, Dr., Rudolf-Breitscheid Strasse 61, D-4000 Düsseldorf 13 (DE)**
Erfinder: **Schröder, Christiane, Hermann-von-Endt-Strasse 3, D-4000 Düsseldorf 13 (DE)**

**Beschreibung**

Die vorliegende Erfindung betrifft Antacida, die zeolithische Alkalialumosilikate der Molekularsiebtypen A, X, Y oder $P_c$ oder deren Gemische in ihren Natrium- und Kaliumformen enthalten. Die Erfindung betrifft ferner die Verwendung zeolithischer Alkalialumosilikate der Molekularsiebtypen A, X, Y oder $P_c$ oder deren Gemisch in ihren Natrium- und Kaliumformen zur Herstellung von Antacida.

Antacida sind Arzneimittel, die überschüssig produzierte Magensäure abbinden. Sie sind indiziert bei durch Übersäuerung bedingten Magenerkrankungen und mit Hyperacidität einhergehendem Ulcus ventriculi oder Ulcus duodeni. Weitere Indikationen sind: Sodbrennen, saures Aufstoßen, Völlegefühl, Magenübersäuerung, Refluxösophagitis, Schwangerschaftssodbrennen, Magenbeschwerden nach Alkohol- und Nikotinmißbrauch sowie nach Diätfehlern, Hiatushernie, Colitis, Therapie und Prophylaxe des «Streß-Ulcus», Gastrophatia neurogenika, medikamentös bedingte Übersäuerung sowie «verdorbener» Magen.

Antacida sind daher nicht nur in der Ulcus-Therapie von besonderer Bedeutung, sie sind die meistverordneten Medikamente überhaupt, sind jedoch überwiegend rezeptfrei erhältlich. Als exemplarischer Stand der Technik seien genannt: W. Leetsch, Deutsche Apothekerzeitung 114, 1307 (1974) «Antacida und das Problem ihrer Wirksamkeitsbeurteilung»; H. Ruppin, Med. Klin. 70, 1237 (1975) «Theoretische und klinische Aspekte der Antacidatherapie»; K.H. Holtermüller, E. Bohlen, M. Castro und H.J. Weis, Med. Klin. 72, 1229 (1977) «Überlegungen zur Therapie mit Antacida»; H. Brunner, E. Penner und G. Grabner, Öst. Ärztetg. 32/1, 27 (1977) «Sind Antacida Plazebos?» R. Güller, Schweiz. med. Wschr. 107, 807 (1977) «Pufferkapazität und Kosten der flüssigen Antazida 1977»; S.B. Siskin, University of Michigan, Medical Center Journal 40, 93 (1974) «Over the Counter Drug Review: Antacid Products»; J. Schnekenburger, Arzneim.-Forsch. (Drug. Res.) 24, 142 und 275 (1975) «In-vitro-Untersuchungen an Antacida»; J. Schnekenburger, loc. cit. 31 (II), 1286 (1981) «In-vitro-Untersuchungen an Antazida».

Dieser Stand der Technik enthält Angaben über die chemische Wirkung der Antacida, zählt Vor- und Nachteile marktgängiger Präparate auf, beschreibt galenisch Formen, Beurteilungskriterien, Kosten-/Nutzen-Relationen und Methoden zu in-vitro-Untersuchungen von Antacida.

Die gebräuchlichsten antacidischen Wirkstoffe sind demnach Natriumhydrogencarbonat, Calciumcarbonat, Magnesiumcarbonat, Aluminiumhydroxid, Aluminiumphosphat, Magnesiumtrisilikat, Aluminiummagnesiumtrisilikat-Hydrat oder Natriumaluminiumsilikat. Die meisten dieser Wirkstoffe (wenn nicht gar alle) besitzen unerwünschte Nebenwirkungen, die besonders bei regelmäßiger Einnahme von Bedeutung sind: Natriumhydrogencarbonat reagiert sehr schnell mit der Magensäure unter Bildung von Kohlendioxid (störende Gasproduktion); überschüssiges Na-Hydrogencarbonat wird resorbiert (besonders bedenklich bei Hypertonikern) und verschieb das Säure-Basen-Gleichgewicht zur alkalischen Seite (dadurch Alkalose, Milch-Alkali-Syndrom). Durch alkalische Reaktion im Magen infolge von überschüssigem Hydrogencarbonat erfolgt erneute kompensatorische Säureproduktion.

Dieser Effekt tritt auch bei Einnahme von Calciumcarbonat ein. Calciumcarbonat bewirkt zusätzlich bereits während der Neutralisationsphase eine Steigerung der Freisetzung von Gastrin, das dann seinerseits die Säuresekretion stimuliert. Vor allem bei wiederholter Anwendung gelangen beträchtliche Ca-Mengen zur Resorption und können zu einer Nierencalcinose führen.

Magnesiumsalze haben eine laxierende Wirkung. Wegen Kumulationsgefahr sind sie bei Nierenfunktionsstörungen kontraindiziert.

Aluminiumsalze als Reaktionsprodukte aluminiumhaltiger Antacida bewirken bei längerer Einnahme (2–3 Wochen) eine Phosphatverarmung. Außerdem wird die Magenentleerung durch Hemmung der Kontraktion der glatten Muskulatur verzögert; es kann eine leichte Verstopfung auftreten.

Bei antacidischen Silikaten treten die gleichen Nebenwirkungen auf wie bei Magnesium- und Aluminiumsalzen. Allgemein wird die Behandlung mit diesen Antacida jedoch als gefahrlos betrachtet. Die antacidischen Silikate binden Säure und belassen eine gewisse Restacidität.

Die DE-A-2 029 265 besceibt ein antacides Gemisch, das dadurch gekennzeichnet ist, daß es Natriumaluminiumsilikat und Milchpulver enthält. Dieses Natriumaluminiumsilikat ist jedoch kein zeolithisches Natriumalumosilikat, wie aus dem Folgenden hervorgeht.

Der Begriff «Natriumalaminiumsilikat» ist zwar ein Sammelbegriff für alle Produkte des Typs $x$ $Na_2O \cdot y\,Al_2O_3 \cdot z\,SiO_2$ für x, y, z, 0, schließt nach strenger Definition jedoch solche Verbindungen aus, in denen das Aluminium in Form eines $AlO_4$-Tetraeders – über Sauerstoff-Brücken also – mit den Silikat-Tetraedern verknüpft ist. Gängigen Lehrbüchern der anorganischen Chemie wie beispielsweise denen von Remy (12. Aufl., Bd. I, S. 615, 1965) oder Holleman-Wiberg (81.–90. Aufl., S. 553–554, 1976) ist zu entnehmen, daß die letzteren, tetraederverknüpften Verbindungen abweichend als «Natriumalumosilikate» bezeichnet werden. Der Gmelin (XI', XII') unterscheidet ebenfalls zwischen «Natriumaluminiumsilikaten» und «Zeolithen».

Die DE-A-2 029 265 schließt demnach zeolithische Antacida aus. Dies wird durch eine Aussage in dieser DE-A- auf S. 3/4 im Beschreibungsteil «Unabhängig von dem gewählten Weg der oralen Einnahme schmeckt das Aluminiumnatriumsilikat sehr schlecht und die Einnahme ist höchst unangenehm» bewiesen, da einen «unangenehmen» Geschmack allenfalls Natriumaluminiumsilikate wie oben definiert mit nicht tetraedrisch von Sauerstoff umgebenen $Al^{3+}$-Kationen aufweisen. Zeolithische Natriumalumosilikate sind geschmacksneutral.

Die DD-A-103 429 beschreibt ein Verfahren zur Herstellung eines Natriumalumosilikat-Gels, das der Zusammensetzung $Na_2O \cdot Al_2O_3 \cdot 2SiO_2 \cdot aq$ entspricht und antacide Eigenschaften besitzt. Auf S. 2, linke Spalte dieser DD-A-103 429 werden als Stand der Technik Molekularsiebe (Zeolithe) mit ausgeprägter Kristallinität beschrieben und ausgeführt, daß diese Zeolithe zwar gewisse säurebindenden Eigenschaften besitzen, die aber für eine pharmazeutische Verwendung als Antacida nicht ausreichen. Deshalb werden in der DD-A-103 429 andere Verbindungen als Zeolithe, nämlich Natriumalumosilikat-Gele hergestellt und als Antacida für geeignet beschrieben. Aus diesen Ausführungen erhielt der Fachmann ein Vorurteil gegen die Verwendung von zeolithischen – und damit kristallinen – Natriumalumosilikaten als Antacida.

Schließlich wurden Vergleichsweise gegenüber der DD-A-103 429 durchgeführt, auf die weiter unten noch eingegangen wird.

Gemäß der JP-A-53/20 437 lassen sich Zeolithe zur Prophylaxe und Therapie von Magengeschwüren bei Schweinen verwenden. Die Zeolithe werden beispielsweise in granulierter oder pulverisierter Form dem, Schweinefutter beigefügt. Bei den hier angesprochenen Zeolithen handelt es sich um solche, die in der Natur vorkommen.

Aufgabe der vorliegenden Erfindung ist es demgegenüber, einerseits Antacida zur Verfügung zu stellen, die bei guten Puffereigenschaften die vorerwähnten Nachteile der Antacida des Standes der Technik und insbesondere die Freigabe physiologisch bedenklicher Ionen nicht aufweisen, sowie andererseits Verbindungen zur Verfügung zu stellen, die bei der Herstellung von verbesserten Antacida verwendet werden können.

Dementsprechend betrifft die Erfindung Antacida auf Basis von Alkalimetallaluminiumsilikaten, die dadurch gekennzeichnet sind, daß sie zeolithische Natriumalumosilikate und/oder Kaliumalumosilikate der Molekularsiebtypen A, X, Y oder $P_c$, die ein Calciumbindevermögen (CaBV) von oberhalb 70 mg CaO/g Aktivsubstanz aufweisen, oder deren Gemische zusammen mit üblichen Füllstoffen und Hilfsstoffen enthalten.

Ferner betrifft die Erfindung die Verwendung von zeolithischen Natriumalumosilikaten und/oder Kaliumalumosilikaten der Molekularsiebtypen A, X, Y oder $P_c$, mit einem Calciumbindevermögen (CaBV) von oberhalb 70 mg CaO/g Aktivsubstanz, oder deren Gemische zur Herstellung von Antacida.

Bevorzugt werden Zeolithe, die ein hohes Kationenaustauschvermögen aufweisen. Dieses Kationenaustauschvermögen läßt sich in Form des «Calciumbindevermögens» (Ca BV) definieren und wird wie folgt ermittelt:

Als Maß für das Kationenaustauschvermögen der Zeolithe wird das Calciumbindevermögen von 1 g Natriumalumosilikat (Aktivsubstanz = AS) pro Liter bei einer Ausgangshärte von 30°d (Deutsche Härte) herangezogen. Zur Bestimmung des Calciumbindevermögens wird 1 l einer wäßrigen, 0,594 g $CaCl_2$ (entsprechend 300 mg CaO/l =

30°d) enthaltenden Lösung mit verdünnter Natronlauge auf einen pH-Wert von 10 eingestellt und mit 1 g AS versetzt. Die gebildete Suspension wird anschließend für die Dauer von 15 Minuten bei einer Temperatur von $22 \pm 2\,°C$ kräftig gerührt. Nach Abfiltrieren des Feststoffes wird die Resthärte X im Filtrat durch komplexometrische Titration mittels Ethylendiamintetraessigsäure ermittelt. Das Calciumbindevermögen in mg CaO/g AS errechnet sich hieraus nach der Formel $(30 - X) \cdot 10$.

Bevorzugt werden ferner Molekularsiebtypen A, X und Y sowie $P_c$ der Typen NaA, NaX, NaY, $NaP_c$, KA, KX, KY sowie $KP_c$ sowie deren Gemische. Die chemischen Zusammensetzungen der Molekularsiebe des Typen NaA bzw. KA entsprechen den Summenformeln

$$1 \pm 0,2 \; Na_2O \cdot 1 \; Al_2O_3 \cdot 2 \pm 0,5 \; SiO_2 \cdot 0 \text{ bis } 6 \; H_2O$$

beziehungsweise

$$1 \pm 0,2 \; K_2O \cdot 1 \; Al_2O_3 \cdot 2 \pm 0,5 \; SiO_2 \cdot 0 \text{ bis } 6 \; H_2O,$$

die der silikatreicheren Molekularsiebe NaX bzw. KX den Formeln

$$0,9 \pm 0,2 \; Na_2O \cdot 1 \; Al_2O_3 \cdot 2,5 \pm 0,5 \; SiO_2 \cdot 0 \text{ bis } 8 \; H_2O$$

beziehungsweise

$$0,9 \pm 0,2 \; K_2O \cdot 1 \; Al_2O_3 \cdot 2,5 \pm 0,5 \; SiO_2 \cdot 0 \text{ bis } 8 \; H_2O,$$

die der noch silikatreicheren Molekularsiebe NaY bzw. KY der Formeln

$$0,9 \pm 0,2 \, Na_2O \cdot 1 \; Al_2O_3 \cdot 5,0 \pm 2,5 \; SiO_2 \cdot 0 \text{ bis } 8 \; H_2O$$

beziehungsweise

$$0,9 \pm 0,2 \; K_2O \cdot 1 \; Al_2O_3 \cdot 5,0 \pm 2,5 \; SiO_2 \cdot 0 \text{ bis } 8 \; H_2O.$$

Diese drei Typen weisen eine sehr ähnliche Struktur des Alumosilikat-Gitters auf.

Die chemische Zusammensetzung des Molekularsiebes $P_c$ das auch als «Zeolith $P_c$» oder «Molekularsieb B» bezeichnet wird, entspricht für den Typ $NaP_c$ bzw. $KP_c$ den Summenformeln

$$0,9 \pm 0,2 \; Na_2O \cdot 1 \; Al_2O_3 \cdot 4 \pm 1,3 \; SiO_2 \cdot 0 \text{ bis } 6 \; H_2O$$

beziehungsweise

$$0,9 \pm 0,2 \; K_2O \cdot 1 \; Al_2O_3 \cdot 4 \pm 1,3 \; SiO_2 \cdot 0 \text{ bis } 6 \; H_2O.$$

Das Röntgenbeugungsdiagramm des Molekularsiebes NaA ist beispielsweise in den DE-B-1 038 015 und 1 038 017, das des Molekularsiebes NaX in der DE-B-1 038 016 und das des Molekularsiebs NaY in der DE-C-1 203 239 beschrieben.

Das Röntgenbeugungsdiagramm des $NaP_c$ ist beispielsweise bei D. W. Breck «Zeolite Molecular Sieves», New York 1974, S. 365, aufgeführt.

Nach einer von Schnekenburger, loc. cit., beschriebenen in-vitro-Titrationsmethode, auf die in den Beispielen eingegangen wird, wurden mit den erfindungsgemäß eingesetzten Zeolithen bei Dosierungen von ca. 300 bis 2000 mg Pufferzeiten von 60 bis 90 Minuten gefunden. Demgegenüber wurden mit verschiedenen amorphen Alkalialumosilikaten, Zeolith $P_t$ (tetragonale Form), Hydrosodalith und ähnlichen, strukturverwandten Alumosilikaten signifikant schlechtere Pufferergebnisse erzielt. Bei diesen Tests schnitten auch unterschiedliche NaMg-Silikate mit Schichtstruktur sowie Natriumsilikate (Metasilikate, Kanemit, Magadiit) überwiegend schlecht ab. Nur Hectorit zeigte ein als Antacidum nutzbares Pufferverhalten, kommt aber aufgrund seiner chemischen Zusammensetzung (lithium-/fluoridhaltiges NaMg-Silikat) als Antacidum aus medizinischer Sicht (s. Nebenwirkungen von Mg-Salzen) nicht infrage.

Das angewendete in-vitro-Verfahren zeigte auch, daß neben den Na-Formen der erfindungsgemäß eingesetzten Zeolith-Typen auch deren K-Formen ohne Wirkungseinbuße als Antacida verwendbar sind. Dies läßt sich medizinisch vorteilhaft nutzen, da durch (teilweise) Verwendung der K-Typen sich nicht nur eine physiologisch unbedenkliche Konzentration an – resorbierbaren – Na-Ionen im Magen-Darm-Trakt erzielen läßt, sondern sogar das physiologisch günstigste Ionenverhältnis $Na^+/K^+$ einstellen läßt. Zahlenmäßige Angaben für ein derartiges günstiges Mischungsverhältnis von Natrium- und Kaliumtypen der erfindungsgemäß eingesetzten zeolithischen Antacida können der Arbeit von W. Siegenthaler, «Klinische Pathophysiologie», Stichwort «Wasser- und Elektrolythaushalt», Thieme 1976, S. 197–201 entnommen werden.

Die in den Beispielen der DD-A-103 429 beschriebenen und entsprechend hergestellten röntgenamorphen Natriumalumosilikate zeigten bei der in-vitro-Titration ungünstige Pufferergebnisse.

Die im Handel befindlichen Präparate «Ulgastrin»⁽ᴵᴱ und «Sulfredox»⁽ᴵᴱ enthalten gemäß Firmenangaben «Natriumaluminiumsilikat». Um dieses identifizieren zu können, wurden Muster dieser beiden Antacida verrieben und mit heißem Wasser behandelt. Der getrocknete Rückstand wurde röntgenographisch untersucht. Zeolithische Strukturen konnten in beiden Fällen nicht nachgewiesen werden. Das Vorliegen der erfindungsgemäß eingesetzten Zeolith-Typen A, X, Y oder $P_c$ in diesen Handelspräparaten ist definitiv auszuschließen. Die in-vitro-Titration-Untersuchungen erbrachten nur bei «Sulfredox» gute Pufferungswerte.

Die Erfindung wird im Folgenden anhand von Beispielen erläutert.

Beispiele

Nach Schnekenburger (loc. cit. 31 (II), 1286 (1981)) wurde zu einer Vorlage aus künstlichem Magensaft und Wasser bei 37°C die zu prüfende Dosis des Antacidums gegeben und die pH-Änderung der resultierenden Suspension in Abhängigkeit von der Zeit registriert. 10 Minuten nach Zugabe des Antacidums wurde weiterer künstlicher Magensaft zudosiert, nach weiteren 10 Minuten das dem Reaktionsgemisch zudosierte Volumen (also Suspension volumengleich dem zudosierten künstlichen Magensaft) entnommen. Diese Entnahme wurde alle 10 Minuten wiederholt, bis der pH-Wert unter 3,0 absank. Dosierung und Entnahme wurden automatisch gesteuert. Künstlicher Magensaft (Säurekonzentration 0,084 Mol/Liter) wurde durch Lösen von 0,32 g Pepsin RN und 0,2 g Natriumchlorid R unter Zusatz von 2,8 ml 3N Salzsäure auf insgesamt 100 ml gemäß DAB 8 (S. 74, Stichwort «Pepsin-Lösung») frisch hergestellt.

Als Geräte wurden verwendet: Autobürette ABU 12, Dosimat Metrohm E 415, Umwälzthermostat Haake FY, pH-Meter CG 811 mit Glaselektrode N 61, Schreiber Servopor Z 10 sowie thermostatisierbares Meßgefäß mit Magnetrührer.

Zur automatischen Titration wurden 20,0 ml künstlicher Magensaft auf 20,0 ml destilliertes Wasser in das Meßgerät eingefüllt. Nach Erreichen der Meßtemperatur von 37°C wurde unter Rühren die zu prüfende Dosis (hier definitionsgemäß ¹/₅ der Einzeldosis) Antacidum in das Meßgerät gegeben und gleichzeitig der Schreiber gestartet. Nach 10 Minuten wurde die Zudosierung von künstlichem Magensaft eingeschaltet und nach weiteren 10 Minuten mit der automatischen Entnahme von 5,0 ml Reaktionsgemisch begonnen.

Mit dem Versuchsablauf wurde eine Pufferungszeit von 60 bis 90 Minuten angestrebt. Hierzu wurden die Antacida in entsprechenden Dosierungen verwendet. Unterschiede zwischen den Pufferungskapazitäten werden dadurch deutlich.

Chemische Zusammensetzung der auf antacide Wirksamkeit untersuchten Alkalialumosilikate

| Bezeichnung | Chemische Zusammensetzung bez. $Al_2O_3 = 1,0$ [a] | | | % $H_2O$ [b] | Ca BV [c] | Anmerkungen |
| --- | --- | --- | --- | --- | --- | --- |
| | $Na_2O$ | $K_2O$ | $SiO_2$ | | | |
| Zeolith NaA | 1,0 | – | 2,0 | 20,0 | 170 | Typ HAB A 40 der Fa. DEGUSSA |
| Zeolith KA | 0,1 | 0,9 | 2,0 | 15,0 | 135 | HAB A 40 mit $K^+$ ausgetauscht [d] |
| Zeolith NaX | 1,0 | – | 2,5 | 12,5 | 120 | Typ 13 X der Fa. UCC |
| Zeolith NaY | 1,0 | – | 3,1 | 24,3 | 100 | Laborprodukt |
| Zeolith $P_c$ | 1,0 | – | 3,3 | 12,0 | 130 | dto. |
| Zeolith $P_t$ | 1,1 | – | 3,7 | 15,5 | 10 | dto. |
| Hydrosodalith | 1,1 | – | 2,0 | 9,1 | 5 | dto. |
| amorphes | 0,7 | – | 2,0 | 16,1 | 140 | Herstellung gem. DD-A-103 429/ |
| NaAl-Silikat | 0,4 | – | 2,1 | 19,5 | 7 | Bsp. 1 dto., Beispiel 2 |

[a] röntgenfluoreszenzanalytisch bestimmt
[b] Glühverlust nach 30 Minuten/800°C
[c] vgl. Bestimmungsvorschrift auf s. 6
[d] mit verd. KCl-Lösung 3mal behandelt; Produkt enthält etwa 90% KA und 10% NaA

Die in den Beispielen für die Titrationsversuche verwendeten Alumosilikate sind bezüglich ihrer chemischen Zusammensetzung, ihrer Herstellung und ihrer Herkunft in der Tabelle beschrieben.

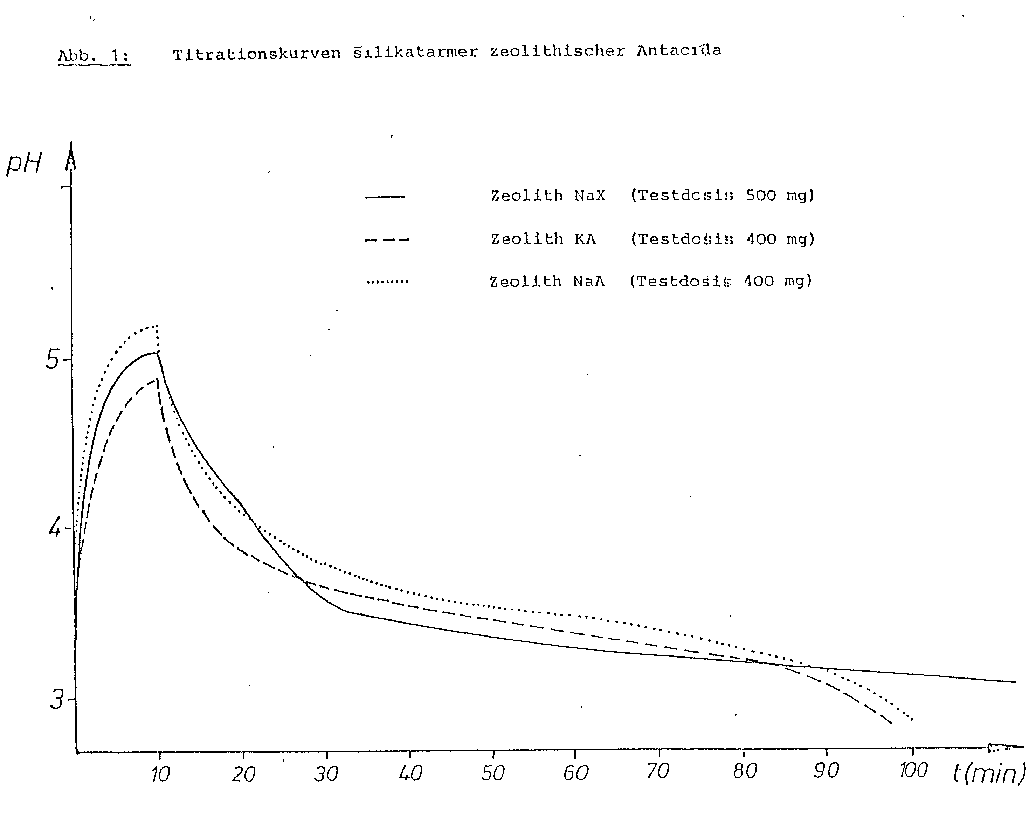

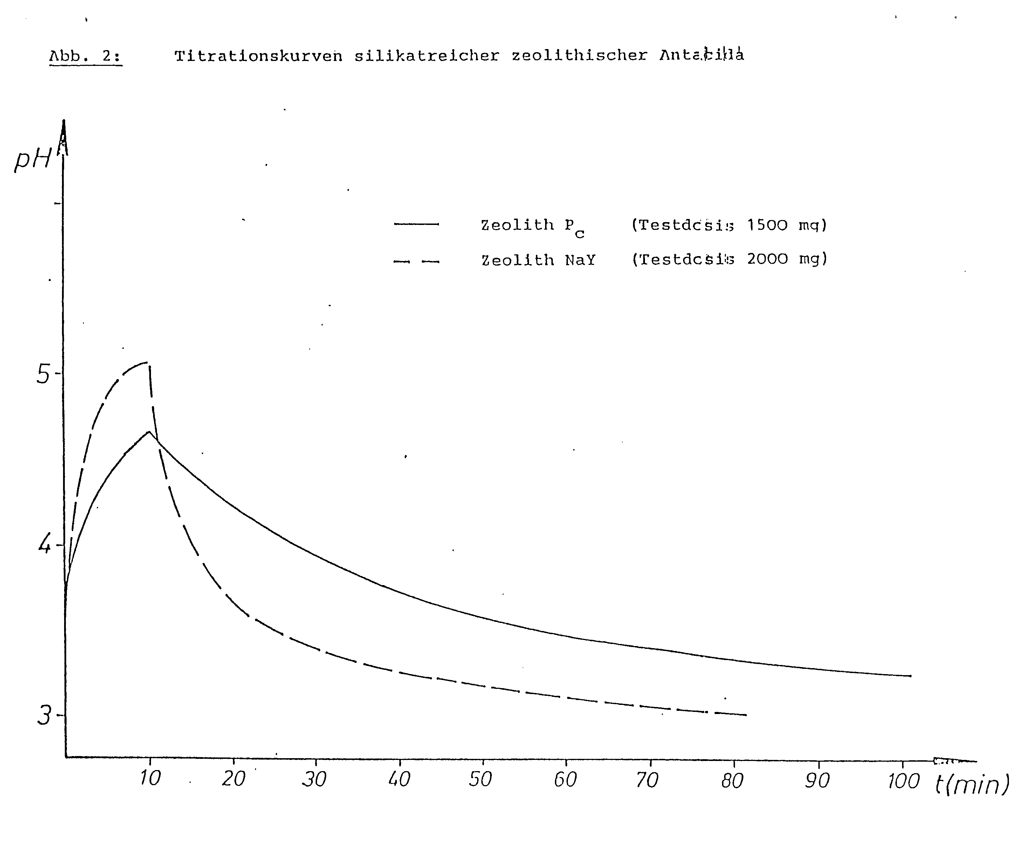

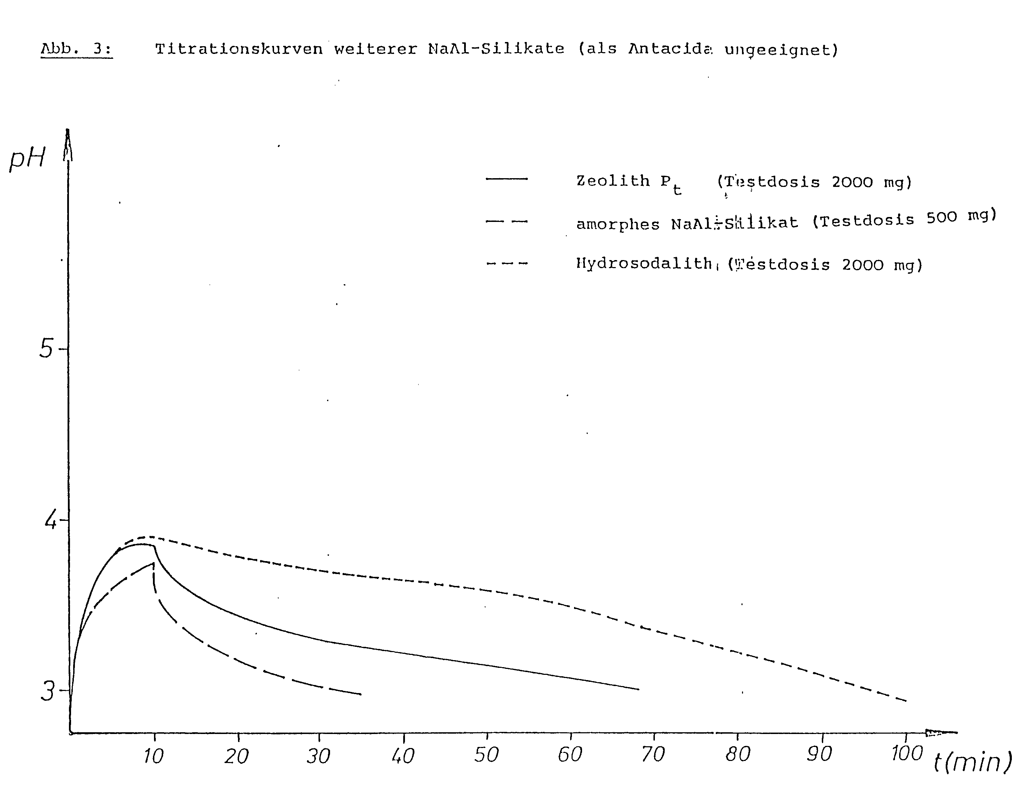

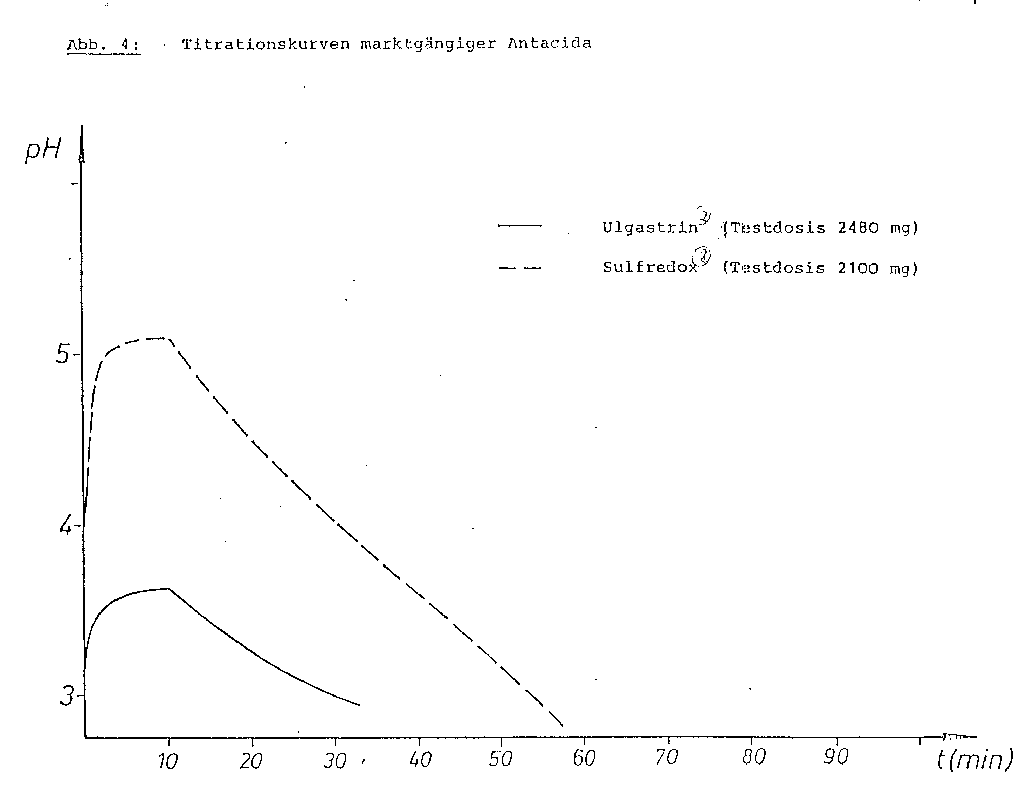

In den Abbildungen 1–4 sind die Titrationskurven der Beispiele dargestellt. Die Abbildungen 1 und 2 zeigen die Titrationskurven der erfindungsgemäß eingesetzten Zeolithe, während die Abbildungen 3 und 4 die Titrationskurven aus dem Stand der Technik zeigen.

Die Beispiele 1 und 2 der DD-A-103 429 wurden nachgearbeitet und die erhaltenen Produkte in der angegebenen Weise titriert, wobei bei der Testdosis von 500 mg die in Abb. 3 (amorphes NaAl-Silikat) dargelegte Titrationskurve erhalten wurde, die ein schlechtes Pufferverhalten aufweist. Durch Änderung der Dosierung (Testdosis 300–2000 mg) konnte keine demgegenüber bemerkenswert geänderte oder gar günstigere Kurve (mit erwünschtem Maximum bei pH ungefähr 5) erzielt werden.

In Abb. 4 werden die Titrationskurven der Handelsprodukte Ulgastrin[®] und Sulfredox[®] aufgeführt. Dabei zeigt sich, daß nur das Sulfredox[®] eine einigermaßen brauchbare Kurve ergibt, die allerdings nur zu einer kürzeren Pufferdauer führt als die erfindungsgemäß eingesetzten Zeolithe.

Aus den Artikeln von Schnekenburger loc. cit. ist zu entnehmen, daß bei vorgesehener Dosierung und entsprechender Testdosis der Maximalwert der Titrationskurven nach 10 Minuten Laufzeit bei pH = 5 liegen soll. Der pH ungefähr = 3,0, entsprechend der normalen Acidität des Magens, soll sich nach höchstens 60 Minuten einstellen, wobei natürlich ein langsameres Abfallen des pH-Wertes entsprechend einer längeren Pufferzeit bevorzugt wird.

Die gemäs vorliegender Erfindung einzusetzenden Zeolithe werden zusammen mit üblichen Füllstoffen, Hilfsstoffen und sonstigen in der Galenik üblichen Zusätzen zu entsprechenden Arzneimitteln formuliert. Die Herstellung ist dem Fachmann bekannt und umfaßt die allgemein gängigen galenischen Anwendungsformen wie Suspensionen, Pillen, Tabletten und insbesondere Pulver.

**Patentansprüche**

1. Antacida auf Basis von Alkalimetallaluminiumsilikaten, dadurch gekennzeichnet, daß sie synthetische zeolithische Natriumalumosilikate und/oder Kaliumalumosilikate der Molekularsiebtypen A, X, Y oder $P_c$, die ein Calciumbindevermögen (CaBV) von oberhalb 70 mg CaO/g Aktivsubstanz aufweisen, oder deren Gemische zusammen mit üblichen Füllstoffen und Hilfsstoffen enthalten.

2. Antacida nach Anspruch 1, dadurch gekennzeichnet, daß sie zeolithische Molekularsiebe des Typs NaA und/oder des Typs KA der Summenformeln

$$1 \pm 0{,}2 \; Na_2O \cdot 1 \; Al_2O_3 \cdot 2 \pm 0{,}5 \; SiO_2 \cdot 0 \; bis \; 6 \; H_2O$$
beziehungsweise
$$1 \pm 0{,}2 \; K_2O \cdot 1 \; Al_2O_3 \cdot 2 \pm 0{,}5 \; SiO_2 \cdot 0 \; bis \; 6 \; H_2O,$$

oder deren Gemische enthalten.

3. Antacida nach Anspruch 1, dadurch gekennzeichnet, daß sie zeolithische Molekularsiebe des Typs NaX und/oder KX der Summenformeln

$$0{,}9 \pm 0{,}2 \; Na_2O \cdot 1 \; Al_2O_3 \cdot 2{,}5 \pm 0{,}5 \; SiO_2 \cdot 0 \; bis \; 8 \; H_2O$$
beziehungsweise
$$0{,}9 \pm 0{,}2 \; K_2O \cdot 1 \; Al_2O_3 \cdot 2{,}5 \pm 0{,}5 \; SiO_2 \cdot 0 \; bis \; 8 \; H_2O,$$

oder deren Gemische enthalten.

4. Antacida nach Anspruch 1, dadurch gekennzeichnet, daß sie zeolithische Molekularsiebe des Typs NaY und/oder KY der Summenformeln

$$0{,}9 \pm 0{,}2 \; Na_2O \cdot 1 \; Al_2O_3 \cdot 5{,}0 \pm 2{,}5 \; SiO_2 \cdot 0 \; bis \; 8 \; H_2O$$
beziehungsweise
$$0{,}9 \pm 0{,}2 \; K_2O \cdot 1 \; Al_2O_3 \cdot 5{,}0 \pm 2{,}5 \; SiO_2 \cdot 0 \; bis \; 8 \; H_2O$$

oder deren Gemische enthalten.

5. Antacida nach Anspruch 1, dadurch gekennzeichnet, daß sie zeolithische Molekularsiebe des Typs $NaP_c$ und/oder $KP_c$ der Summenformeln

$$0{,}9 \pm 0{,}2 \; Na_2O \cdot 1 \; Al_2O_3 \cdot 4 \pm 1{,}3 \; SiO_2 \cdot 0 \; bis \; 6 \; H_2O$$
beziehungsweise
$$0{,}9 \pm 0{,}2 \; K_2O \cdot 1 \; Al_2O_3 \cdot 4 \pm 1{,}3 \; SiO_2 \; 0 \; bis \; 6 \; H_2O$$

oder deren Gemische enthalten.

6. Verwendung von synthetischen zeolithischen Natriumalumosilikaten und/oder Kaliumalumosilikaten der Molekularsiebtypen A, X, Y oder $P_c$, mit einem Calciumbindevermögen (CaBV) von oberhalb 70 mg CaO/g Aktivsubstanz, oder deren Gemische zur Herstellung von Antacida.

7. Verwendung nach Anspruch 6, gekennzeichnet durch den Einsatz zeolithischer Molekularsiebe des Typs NaA und/oder des Typs KA der Summenformeln

$$1 \pm 0{,}2 \; Na_2O \cdot 1 \; Al_2O_3 \cdot 2 \pm 0{,}5 \; SiO \cdot 0 \; bis \; 6 \; H_2O$$
beziehungsweise
$$1 \pm 0{,}2 \; K_2O \cdot 1 \; Al_2O_3 \cdot 2 \pm 0{,}5 \; SiO_2 \cdot 0 \; bis \; 6 \; H_2O,$$

oder deren Gemische.

8. Verwendung nach Anspruch 6, gekennzeichnet durch den Einsatz zeolithischer Molekularsiebe des Types NaX und/oder KX der Summenformeln

$$0{,}9 \pm 0{,}2 \; Na_2O \cdot 1 \; Al_2O_3 \cdot 2{,}5 \pm 0{,}5 \; SiO_2 \cdot 0 \; bis \; 8 \; H_2O$$
beziehungsweise
$$0{,}9 \pm 0{,}2 \; K_2O \cdot 1 \; Al_2O_3 \cdot 2{,}5 \pm 0{,}5 \; SiO_2 \cdot 0 \; bis \; 8 \; H_2O,$$

oder deren Gemische.

9. Verwendung nach Anspruch 6, gekennzeichnet durch den Einsatz zeolithischer Molekularsiebe des Typs NaY und/oder KY der Summenformeln

$$0{,}9 \pm 0{,}2 \; Na_2O \cdot 1 \; Al_2O_3 \cdot 5{,}0 \pm 2{,}5 \; SiO_2 \cdot 0 \; bis \; 8 \; H_2O$$
beziehungsweise
$$0{,}9 \pm 0{,}2 \; K_2O \cdot 1 \; Al_2O_3 \cdot 5{,}0 \pm 2{,}5 \; SiO_2 \cdot 0 \; bis \; 8 \; H_2O$$

oder deren Gemische.

10. Verwendung nach Anspruch 6, gekennzeichnet durch den Einsatz zeolithischer Molekularsiebe des Typs $NaP_c$ und/oder $KP_c$ der Summenformeln

$0{,}9 \pm 0{,}2\ Na_2O \cdot 1\ Al_2O_3 \cdot 4 \pm 1{,}3\ SiO_2 \cdot 0$ bis $6\ H_2O$
beziehungsweise
$0{,}9 \pm 0{,}2\ K_2O \cdot 1\ Al_2O_3 \cdot 4 \pm 1{,}3\ SiO_2 \cdot 0$ bis $6\ H_2O$

oder deren Gemische.

## Claims

1. Antacids based on alkali metal aluminium silicates, characterized in that they contain synthetic sodium alumosilicates and/or potassium alumosilicates of A, X, Y or $P_c$ molecular sieves, which have a calcium binding power (CaBP) above 70 mg CaO/g active substance, or mixtures thereof together with standard fillers and auxiliaries.

2. Antacids as claimed in Claim 1, characterized in that they contain zeolitic molecular sieves of the NaA and/or KA type corresponding to the following summation formulae

$1 \pm 0{.}2\ Na_2O \cdot 1\ Al_2O_3 \cdot 2 \pm 0{.}5\ SiO_2 \cdot 0$ to $6\ H_2O$
and
$1 \pm 0{.}2\ K_2O \cdot 1\ Al_2O_3 \cdot 2 \pm 0{.}5\ SiO_2 \cdot 0$ to $6\ H_2O$

or mixtures thereof.

3. Antacids as claimed in Claim 1, characterized in that they contain zeolitic molecular sieves of the NaX and/or KX type corresponding to the following summation formulae

$0{.}9 \pm 0{.}2\ Na_2O \cdot 1\ Al_2O_3 \cdot 2{.}5 \pm 0{.}5\ SiO_2 \cdot 0$ to $8\ H_2O$
and
$0{.}9 \pm 0{.}2\ K_2O \cdot 1\ Al_2O_3 \cdot 2{.}5 \pm 0{.}5\ SiO_2 \cdot 0$ to $8\ H_2O$,

or mixtures thereof.

4. Antacids as claimed in Claim 1, characterized in that they contain zeolitic molecular sieves of the NaY and/or KY type corresponding to the following summation formulae

$0{.}9 \pm 0{.}2\ Na_2O \cdot 1\ Al_2O_3 \cdot 5{.}0 \pm 2{.}5\ SiO_2 \cdot 0$ to $8\ H_2O$
and
$0{.}9 \pm 0{.}2\ K_2O \cdot 1\ Al_2O_3 \cdot 5{.}0 \pm 2{.}5\ SiO_2 \cdot 0$ to $8\ H_2O$

or mixtures thereof.

5. Antacids as claimed in Claim 1, characterized in that they contain zeolitic molecular sieves of the $NaP_c$ and/or $KP_c$ type corresponding to the following summation formulae

$0{.}9 \pm 0{.}2\ Na_2O \cdot 1\ Al_2O_3 \cdot 4 \pm 1{.}3\ SiO_2 \cdot 0$ to $6\ H_2O$
and
$0{.}9 \pm 0{.}2\ K_2O \cdot 1\ Al_2O_3 \cdot 4 \pm 1{.}3\ SiO_2 \cdot 0$ to $6\ H_2O$

or mixtures thereof.

6. The use of synthetic sodium alumosilicates and/or potassium alumosilicates of A, X, Y or $P_c$ molecular sieves, which have a calcium binding power (CaBP) above 70 mg CaO/g active substance, for the production of antacids.

7. The use claimed in Claim 6, characterized by the use of zeolitic molecular sieves of the NaA and/or KA type corresponding to the following summation formulae

$1 \pm 0{.}2\ Na_2O \cdot 1\ Al_2O_3 \cdot 2 \pm 0{.}5\ SiO_2 \cdot 0$ to $6\ H_2O$
and
$1 \pm 0{.}2\ K_2O \cdot 1\ Al_2O_3 \cdot 2 \pm 0{.}5\ SiO_2 \cdot 0$ to $6\ H_2O$

or mixtures thereof.

8. The use claimed in Claim 6, characterized by the use of zeolitic molecular sieves of the NaX and/or KX type corresponding to the following summation formulae

$0{.}9 \pm 0{.}2\ Na_2O \cdot 1\ Al_2O_3 \cdot 2{.}5 \pm 0{.}5\ SiO_2 \cdot 0$ to $8\ H_2O$
and
$0{.}9 \pm 0{.}2\ K_2O \cdot 1\ Al_2O_3 \cdot 2{.}5 \pm 0{.}5\ SiO_2 \cdot 0$ to $8\ H_2O$,

or mixtures thereof.

9. The use claimed in Claim 6, characterized by the use of zeolitic molecular sieves of the NaY and/or KY type corresponding to the following summation formulae

$0{.}9 \pm 0{.}2\ Na_2O \cdot 1\ Al_2O_3 \cdot 5{.}0 \pm 2{.}5\ SiO_2 \cdot 0$ to $8\ H_2O$
and
$0{.}9 \pm 0{.}2\ K_2O \cdot 1\ Al_2O_3 \cdot 5{.}0 \pm 2{.}5\ SiO_2 \cdot 0$ to $8\ H_2O$,

or mixtures thereof.

10. The use claimed in Claim 6, characterized by the use of zeolitic molecular sieves of the $NaP_c$ and/or $KP_c$ type corresponding to the following summation formulae

$0{.}9 \pm 0{.}2\ Na_2O \cdot 1\ Al_2O_3 \cdot 4 \pm 1{.}3\ SiO_2 \cdot 0$ to $6\ H_2O$
and
$0{.}9 \pm 0{.}2\ K_2O \cdot 1\ Al_2O_3 \cdot 4 \pm 1{.}3\ SiO_2 \cdot 0$ to $6\ H_2O$

or mixtures thereof.

## Revendications

1. Antacides à base d'aluminosilicates de métaux alcalins, caractérisés en ce qu'ils contiennent des aluminosilicates de potassium et/ou des aluminosilicates de sodium synthétiques zéolitiques des types de tamis moléculaires A, X, Y ou $P_c$ qui ont un pouvoir de fixation du calcium (CaBV) de plus de 70 mg de CaO/g de substance active, ou leurs mélanges conjointement avec des substances auxiliaires et des charges habituelles.

2. Antacides selon la revendication 1, caractérisés en ce qu'ils contiennent des tamis moléculaires zéolitiques de type NaA et/ou de type KA répondant aux formules brutes:

$1 \pm 0{,}2\ Na_2O \cdot 1\ Al_2O_3 \cdot 2 \pm 0{,}5\ SiO_2 \cdot 0$ à $6\ H_2O$
ou
$1 \pm 0{,}2\ K_2O \cdot 1\ Al_2O_3 \cdot 2 \pm 0{,}5\ SiO_2 \cdot 0$ à $6\ H_2O$

ou leurs mélanges.

3. Antacides selon la revendication 1, caractérisés en ce qu'ils contiennent des tamis moléculaires zéolitiques de type NaX et/ou KX répondant aux formules brutes:

$0{,}9 \pm 0{,}2 \ Na_2O \cdot 1 \ Al_2O_3 \cdot 2{,}5 \pm 0{,}5 \ SiO_2 \cdot 0 \ \text{à} \ 8 \ H_2O$
ou
$0{,}9 \pm 0{,}2 \ K_2O \cdot 1 \ Al_2O_3 \cdot 2{,}5 \pm 0{,}5 \ SiO_2 \cdot 0 \ \text{à} \ 8 \ H_2O$

ou leurs mélanges.

4. Antacides selon la revendication 1, caractérisés en ce qu'ils contiennent des tamis moléculaires zéolitiques de type NaY et/ou Ky répondant aux formules brutes:

$0{,}9 \pm 0{,}2 \ Na_2O \cdot 1 \ Al_2O_3 \cdot 5{,}0 \pm 2{,}5 \ SiO_2 \cdot 0 \ \text{à} \ 8 \ H_2O$
ou
$0{,}9 \pm 0{,}2 \ K_2O \cdot 1 \ Al_2O_3 \cdot 5{,}0 \pm 2{,}5 \ SiO_2 \cdot 0 \ \text{à} \ 8 \ H_2O$

ou leurs mélanges.

5. Antacides selon la revendication 1, caractérisés en ce qu'ils contiennent des tamis moléculaires zéolitiques de type $NaP_c$ et/ou $KP_c$ répondant aux formules brutes:

$0{,}9 \pm 0{,}2 \ Na_2O \cdot 1 \ Al_2O_3 \cdot 4 \pm 1{,}3 \ SiO_2 \cdot 0 \ \text{à} \ 6 \ H_2O$
ou
$0{,}9 \pm 0{,}2 \ K_2O \cdot 1 \ Al_2O_3 \cdot 4 \pm 1{,}3 \ SiO_2 \cdot 0 \ \text{à} \ 6 \ H_2O$

ou leurs mélanges.

6. Utilisation d'aluminosilicates de potassium et/ou d'aluminosilicates de sodium synthétiques zéolitiques des types de tamis moléculaires A, X, Y, ou $P_c$ ayant un pouvoir de fixation du calcium (CaBV) de plus de 70 mg de CaO/g de substance active, ou leurs mélanges, pour la préparation d'antacides.

7. Utilisation selon la revendication 6, caractérisée par l'utilisation de tamis moléculaires zéolitiques de type NaA et/ou de type KA répondant aux formules brutes:

$1 \pm 0{,}2 \ Na_2O \cdot 1 \ Al_2O_3 \cdot 2 \pm 0{,}5 \ SiO_2 \cdot 0 \ \text{à} \ 6 \ H_2O$
ou
$1 \pm 0{,}2 \ K_2O \cdot 1 \ Al_2O_3 \cdot 2 \pm 0{,}5 \ SiO_2 \cdot 0 \ \text{à} \ 6 \ H_2O$

ou leurs mélanges.

8. Utilisation selon la revendication 6, caractérisée par l'utilisation de tamis moléculaires zéolitiques de type NaX et/ou KX répondant aux formules brutes:

$0{,}9 \pm 0{,}2 \ Na_2O \cdot 1 \ Al_2O_3 \cdot 2{,}5 \pm 0{,}5 \ SiO_2 \cdot 0 \ \text{à} \ 8 \ H_2O$
ou
$0{,}9 \pm 0{,}2 \ K_2O \cdot 1 \ Al_2O_3 \cdot 2{,}5 \pm 0{,}5 \ SiO_2 \cdot 0 \ \text{à} \ 8 \ H_2O$

ou leurs mélanges.

9. Utilisation selon la revendication 6, caractérisée par l'utilisation de tamis moléculaires zéolitiques de type NaY et/ou KY répondant aux formules brutes:

$0{,}9 \pm 0{,}2 \ Na_2O \cdot 1 \ Al_2O_3 \cdot 5{,}0 \pm 2{,}5 \ SiO_2 \cdot 0 \ \text{à} \ 8 \ H_2O$
ou
$0{,}9 \pm 0{,}2 \ K_2O \cdot 1 \ Al_2O_3 \cdot 5{,}0 \pm 2{,}5 \ SiO_2 \cdot 0 \ \text{à} \ 8 \ H_2O$

ou leurs mélanges.

10. Utilisation selon la revendication 6, caractérisée par l'utilisation de tamis moléculaires zéolitiques de type $NaP_c$ et/ou $KP_c$ répondant aux formules brutes:

$0{,}9 \pm 0{,}2 \ Na_2O \cdot 1 \ Al_2O_3 \cdot 4 \pm 1{,}3 \ SiO_2 \cdot 0 \ \text{à} \ 6 \ H_2O$
ou
$0{,}9 \pm 0{,}2 \ K_2O \cdot 1 \ Al_2O_3 \cdot 4 \pm 1{,}3 \ SiO_2 \cdot 0 \ \text{à} \ 6 \ H_2O$

ou leurs mélanges.

Abb. 1:   Titrationskurven silikatarmer zeolithischer Antacida

pH

Zeolith NaX   (Testdosis 500 mg)

Zeolith KA   (Testdosis 400 mg)

Zeolith NaA   (Testdosis 400 mg)

5

4

3

10   20   30   40   50   60   70   80   90   100   t(min)

EP 0 100 070 B1

Abb. 2: Titrationskurven silikatreicher zeolithischer Antacida

Zeolith P_C (Testdosis 1500 mg)

Zeolith NaY (Testdosis 2000 mg)

Abb. 3: Titrationskurven weiterer NaAl-Silikate (als Antacida ungeeignet)

Zeolith $P_t$ (Testdosis 2000 mg)

amorphes NaAl-Silikat (Testdosis 500 mg)

Hydrosodalith (Testdosis 2000 mg)

Abb. 4:  Titrationskurven marktgängiger Antacida

Legend:
——— Ulgastrin[3] (Testdosis 2480 mg)
— — Sulfredox[3] (Testdosis 2100 mg)

EP 0 100 070 B1